# EUROPÄISCHE PATENTSCHRIFT

(11) **EP 0 854 877 B1**
(45) Veröffentlichungstag und Bekanntmachung des Hinweises auf die Patenterteilung: **16.01.2002**
(21) Anmeldenummer: 96938002.1
(22) Anmeldetag: 06.09.1996
(51) Int. Cl.: C07F 17/02, C07F 17/00, G01N 33/48, C12Q 1/68, C12N 9/16, C07H 23/00

(54) **METALLOCEN-PHOSPHORAMIDIT-KONJUGATE, VERFAHREN ZU IHRER HERSTELLUNG SOWIE DEREN VERWENDUNG**
METALLOCENE-PHOSPHORAMIDITE CONJUGATES, PROCESS FOR THEIR PREPARATION AND THEIR USE
PRODUITS CONJUGUES METALLOCENES-PHOSPHORAMIDITES, LEUR PROCEDE DE FABRICATION ET LEUR UTILISATION

(30) Priorität: 07.09.1995 DE 19533093
(43) Veröffentlichungstag der Anmeldung: 29.07.1998
(73) Patentinhaber: Deutsches Krebsforschungszentrum Stiftung des öffentlichen Rechts, 69120 Heidelberg (DE)
(72) Erfinder: WIESSLER, Manfred, D-69120 Heidelberg (DE); SCHÜTTE, Dagmar, D-69221 Dossenheim (DE)
(74) Vertreter: Schüssler, Andrea, Dr.
(86) Internationale Anmeldenummer: DE9601681
(87) Internationale Veröffentlichungsnummer: WO9709337

(56) Entgegenhaltungen:
- CHEMICAL COMMUNICATIONS, Nr. 4, 1996, Seiten 555-557, XP002022094 MUCIC, R.C. ET AL.: "synthesis and characterization of dna with ferrocenyl groups attached to their 5'-termini: electrochemical characterization of a redox-active nucleotide monolayer"
- CHEMICAL ABSTRACTS, vol. 121, no. 11, 12.September 1994 Columbus, Ohio, US; abstract no. 134702f, DEBITSUDO, A.: "preparation of oligonucleotide having redox group" XP002022095 & JP 06 041 184 A (MITSUBISHI CHEMICAL INDUSTRIES) 15.Februar 1994

## Beschreibung

Verwendung von Metallocen-Phosphoramidit-Konjugaten zur elektronenmikroskopisch deteketierbaren Markierung von oligonukleotiden bzw. DNA oder RNA

Die vorliegende Erfindung betrifft die Verwendung von Metallocen - Phosphoramidit - Konjugaten zur elektronenmikroskopisch detektierbaren Markierung von Oligonukleotiden bzw, DNA ode RNA.

Bisher gab es Schwierigkeiten Oliganukleotide und Teile von DNA bzw. RNA im Elektronenmikroskop zu detektieren bzw. zu differenzieren. Es gibt bisher keine zufriedenstellende Möglichkeit, DNA bzw. RNA in reproduzierbarer Weise elektronenmikroskopisch sichtbar zu machen, um daran Studien durchführen zu können.

Chemical Abstracts, Vol. 121, No. 11, 12. Sept. 1994, Abstract No. 134702f, beschreibt die Verbindung W(CH₂)₃OP(OCH₂CH₂CN)N(CHMe₂)₂, wobei W Ferrocenyl bedeutet. Diese Verbindung wird zur Herstellung von Oligonukleotiden mit einer Redoxgruppe verwendet, die ihrerseits als Material für Sensoren und Vorrichtungen zur Detektion von Redoxpotentialen nützlich sind.

Aufgabe der vorliegenden Erfindung war es deshalb, ein Mittel bereitzustellen, mit dem durch einfache Veränderung der DNA bzw. RNA, möglichst schon bei der Synthese, ein Signal an der DNA bzw. RNA angebracht werden kann, um die DNA bzw. RNA besser elektronenmikroskopisch nachweisbar zu machen.

Gelöst wird diese Aufgabe durch die Merkmale des Patentanspruchs 1. Vorteilhafte Ausgestaltungen ergeben sich aus den Unteransprüchen. Insbesondere wird die Aufgabe durch die Verwendung von Metallocen - Phosphoramidit - konjugaten gelöst.

Bei dem erfindungsgemäß verwendeten Metallocen-Phosphoramidit-Konjugat handelt es sich um Konjugate von einem oder mehreren Phosphoramiditen an ein oder mehrere Metallocene, bevorzugt Übergangsmetall-Metallocene, insbesondere Ferrocen, Ruthenocen oder Osmocen. Dabei können die in einem Konjugat vorkommenden Metallocene gleich oder verschieden sein. Zwischen dem Phosphoramidit und dem Metallocen befindet sich vorzugsweise noch ein Spacer, z.B. ein C₁-C₁₀-Alkylspacer, bevorzugt eine Propyl- oder Butylgruppe.

Bei dem erfindungsgemäßen Metallocen-Phosphoramidit-Konjugat handelt es sich bevorzugt um Metallocenylalkyl-(2-cyanoethyl)-diisopropylamidophosphit, wobei das Metall ein Übergangsmetall ist, vorzugsweise Fe, Ru oder Os.

Das Metallocen-Phosphoramidit-Konjugat wird vorzugsweise im Rahmen der Oligonukleotidsynthese an ein Festphasen-gekoppeltes Oligomer addiert. Gute Ergebnisse werden erreicht, wenn das Metallocen-Phosphoramidit-Konjugat im Vergleich zu den Oligonukleotiden im Überschuß eingesetzt wird. Bei der Oligonukleotidsynthese kommt es dann zu einer Metallocen-Markierung der DNA bzw. RNA, die elektronenmikroskopisch damit gut untersuchbar wird. Es ist natürlich auch möglich die DNA bzw. RNA nachträglich durch das Konjugat mit dem Metallocen zu markieren. Mit den metallocenmarkierten Oligonukleotiden lassen sich Feinstrukturen von DNA bzw. RNA im Elektronenmikroskop untersuchen. Antisense-Oligonukleotide können auf ihre Hybrdisierungseigenschaften hin untersucht werden, es kann zwischen spezifischen und unspezifischen Bindungen unterschieden werden, die Doppel- oder Tripel-Helix-Bildung kann verfolgt werden. Bei bekannter Sequenz lassen sich Strukturen im Elektronenmikroskop der Sequenz zuordnen. Außerdem kann bei Markierung verschiedener Oligonukleotide mit unterschiedlichen Metallocenen eine bevorzugte Hybridisierung gemessen werden. Mittels der Elektronenverlustspektroskopie kann man dabei einzelne Metallatome anhand ihrer charakteristischen Elektronenspektren nachweisen.

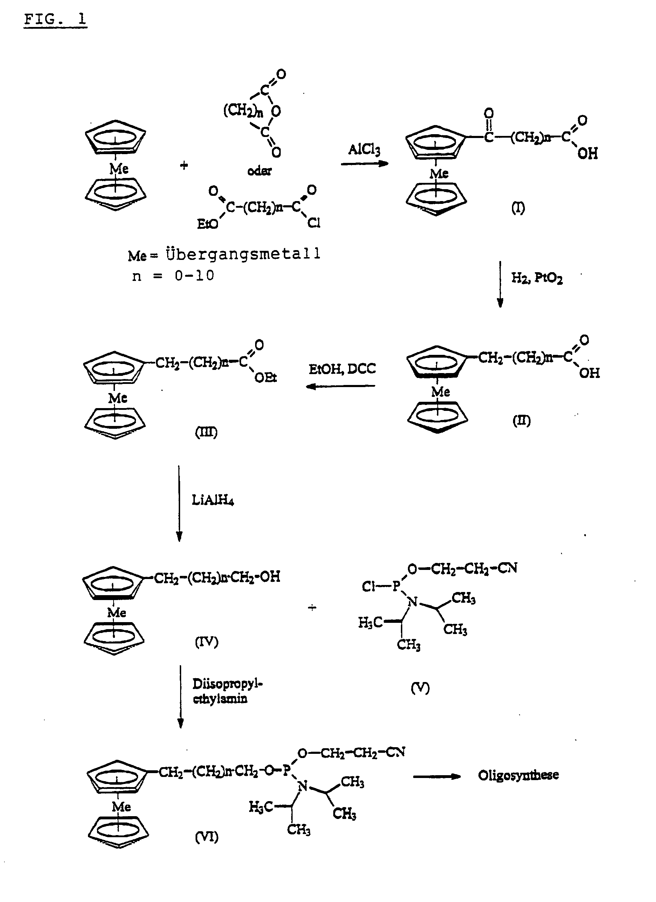

Die Herstellung der Metallocen-Phosphoramidit-Konjugate erfolgt beispielsweise über ein Verfahren, dessen Reaktionsschema in Fig. 1 dargestellt ist: Ein Metallocen (Übergangsmetall, bevorzugt Ferrocen, Ruthenocen oder Osmocen) wird mit einem Dicarbonsäurechlorid oder -anhydrid unter der Katalyse eines Friedel-Crafts-Katalysators zur Metallocenoylalkylcarbonsäure (I) umgesetzt. Die Ketofunktion im Molekül wird danach unter geeigneten Bedingungen hydriert, z.B. Hydrierung unter Katalyse eines PtO₂-Katalysator (II). Die Carbonsäurefunktion im Molekül wird dann durch Umsetzung mit einem Alkohol verestert (III) und anschließend unter geeigneten Bedingungen, z.B. durch Umsetzung mit LiAlH₄, reduziert (IV). Den entstandenen Alkohol (IV) kann man mit einem Phosphoramiditderivat, z.B. dem in der Oligonukleotidsynthese üblichen Chloro-N,N-diisopropylamino-cyanoethoxyphosphit (V), umsetzen. Das erhaltene Konjugat aus Metallocen und Phosphoramidit (VI) kann nun in der Oligonukleotidsynthese eingesetzt werden.

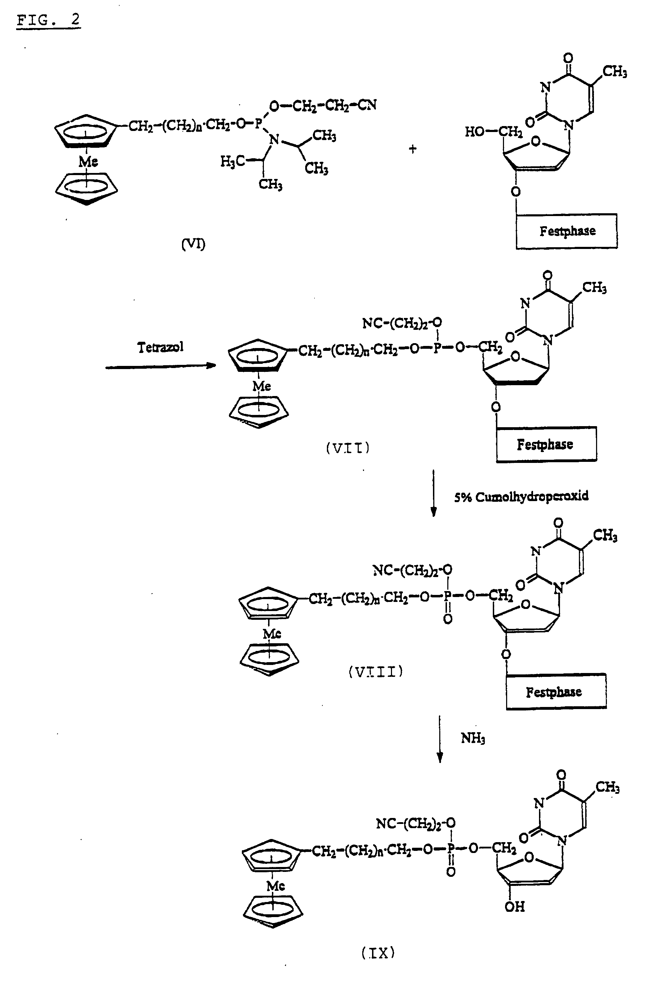

Die Markierung von (Oligo)nukleotiden während ihrer Synthese ist in Fig. 2 gezeigt. Das in Fig. 2 gezeigte Reaktionsschema ist beispielhaft für die durch die Umsetzung von Metallocen-Phosphoramidit-Konjugat mit einem Nukleotid oder Oligonukleotid stattfindende Kopplung des (Oligo)nukleotids an das Metallocen. So wird das Metallocen-Phosphoramidit-Konjugat (VI) mit einem Festphasen-gekoppelten 5'-OH freien (Oligo)nukleotid mit einem Aktivator, z.B. Tetrazol, umgesetzt. Die entstehende Verbindung (VII) wird mit einem Oxidationsmittel, z.B. verdünnter Peroxidlösung, insbesondere 5%iger Cumolhydroperoxidlösung, umgesetzt, wobei eine Verbindung (VIII) entsteht, bei der die vorherige Phosphoramiditgruppierung in eine Phosphatgruppe umgewandelt worden ist. Unter Baseneinwirkung, z.B. Ammoniak, wird das an das Metallocen gekoppelte Nukleotid von der Festphase abgetrennt, wobei Verbindung (IX) entsteht.

Von den Erfindern wurde herausgefunden, daß die durch die Metallocene markierten (Oligo)nukleotide außer der guten Analysierbarkeit im Elektronenmikroskop eine andere interessante Anwendung haben, nämlich als künstliche Restriktionsenzyme. Es ist allgemein bekannt, daß im Körper Fremdstoffe durch Oxidation in eine wasserlösliche Form überführt werden. Das dafür benötigte Wasserstoffperoxid wird aus Sauerstoff zunächst durch Reduktion zu einem Superoxidanion (O₂) hergestellt. Dieses Anion kann mit Hilfe der Superoxid-Dismutase zu Sauerstoff und Wasserstoffperoxid disproportionieren. Sind Eisen(II)-Ionen vorhanden kann das intermediär entstehende H₂O₂ zu Hydroxylradikalen, die eine höchst reaktive Spezies darstellen, abreagieren. Findet diese Reaktion in der näheren Umgebung von DNA statt, kann die DNA ähnlich wie von einem Restriktionsenzym gespalten werden. Führt man die Spaltung künstlich herbei, fügt man ein Reduktionsmittel zu, z.B. Dithiothreitol (DTT), das aus dem Eisen (III) dann wieder Eisen (II) erzeugt, welches erneut reagieren kann. Ferrocen kann diese Reaktion als Eisen (II)-enthaltende Substanz auslösen. Wenn man ein Ferrocen-markiertes Oligonukleotid einsetzt, wird durch die Hybridisierung des Oligonukleotids gegen einzelsträngige DNA (unter Bildung von Watson-Crick-Basenpaarung) oder auch doppelsträngige DNA (unter Bildung von Hoogsteen-Basenpaarung) das Eisen(II)-lon in direkter Umgebung zur DNA an einer bestimmten Sequenz fixiert. Katalysiert dort das Eisen (II) die Spaltung von H₂O₂ kommt es durch die entstehenden reaktiven Hydroxylradikale bei der DNA an einer definierten Stelle zu einem Strangbruch. Idealerweise erfolgt dieses Schneiden nur an einer spezifischen Stelle der DNA. Das Beschriebene gilt natürlich anstelle von Eisen (II) auch für Ruthenium (II) und Osmium (II) in den jeweiligen Ruthenocenen und Osmocenen, sowie für alle Übergangsmetall-Metallocene, in denen das Übergangsmetall einfach durch Reduktion bzw. Oxidation in verschiedene Oxidationsstufen überführt werden kann.

Die Erfindung wird nun weiter mit Bezug auf die Figur erläutert:
- Fig. 1:: beispielhaftes Reaktionsschema für die Herstellung eines Metallocen-Phosphoramidit-Konjugats
- Fig. 2:: Verwendung des Metallocen-Phosphoramidit-Konjugats in der Oligosynthese zur Markierung von Oligonukleotiden mit Metallocenen

Die Erfindung wird nun weiter mit Bezug auf die nachfolgenden Beispiele beschrieben:

### Beispiel 1: Herstellung von (5-Ferrocenylpentyl)-(2-cyanoethyl)-diisopropylamidophosphit

### a) 4-Ferrocenoylbutansäure (I)

Zu 3,5 g (26 mmol) Aluminiumtrichlorid in 100 ml wasserfreiem CH₂Cl₂ wurde eine Mischung aus 5,0 g (27 mmol) Ferrocen und 2,3 g (20 mmol) Glutarsäureanhydrid in 100 ml CH₂Cl₂ getropft. Die Lösung färbt sich dabei violett. Nach 3 h unter Rückfluß wurde der Ansatz hydrolysiert und zur Entfernung von Reaktionsprodukten, die weder in wässriger noch in organischer Phase löslich sind, durch eine Fritte gesaugt. Die wässrige Phase wurde anschließend abgetrennt und die organische Phase dreimal mit Wasser gewaschen, mit NaSO₄ getrocknet und eingeengt.

Ausbeute: 3,5 g (58,3 % d. Th.)
DC:R_{f} (Kieselgel, CHCL₃/MeOH 95:5): 0,6
SC: Alox, sauer (Akt. III), 1) CH₂Cl₂; 2) CH₂Cl₂/MeOH 1:1 + 5 Vol-% HOAc
¹H-NMR (250 MHz) CDCl₃: δ = 4,76-4,75 (t, 2H, Cp subst.); 4,51-4,50 (t, 2H, Cp subst.); 4,19 (s, 5H, Cp unsubst.); 2,82-2,76 (t, 2H, CH₂-C(O)); 2,40-2,34 (t, 2H, CH₂-COO); 1,90-1,86 (m, 2H, C-CH₂-C)
MS: (C₁₅H₁₆FeO₃, 300,04), m/z = 300 (M⁺, 100 %)

### b) 5-Ferrocenylpentansäure (II)

2,0 g (6,66 mmol) 4-Ferrocenoylbutansäure (I) wurden in einem Dreihalskolben mit 50 ml Eisessig gelöst, der anschließend mit Stickstoff gespült wurde. 100 mg Platinoxid wurden als Katalysator zugefügt. Der Kolben wurde abermals mit Stickstoff gespült und dann mit Wasserstoff beladen. Nach 18 h ist die Hydrierung abgeschlossen und der Katalysator wird abgesaugt. Der Reaktionsansatz wird mit 100 ml H₂O verdünnt und 5x mit CH₂Cl₂ extrahiert. Die vereinigten organischen Phasen wurden noch 2x mit H₂O gewaschen, mit NaSO₄ getrocknet, eingeengt und chromatographiert.

Ausbeute: 1,91 g (98 % d. Th.)
DC: R_{f}, (Kieselgel, CHCl₃/MeOH 95:5): 0,65
SC: Alox, sauer (Akt.III), 1) CH₂Cl₂; 2) CH₂Cl₂/MeOH 95:5 + 5 Vol-% HOAc
¹H-NMR (250 MHz) CDCL₃: δ = 4,08 (s, 5H, Cp unsubst.); 4,03 (s, 4H, Cp subst.); 2,37-2,32 (m, 4H, Fc-CH₂, CH₂-COOH); 1,67-1,55 (m, 4H, 2xCH₂)
MS: (C₁₅H₁₈FeO₂, 286,07), m/z = 286 (M⁺, 100%); 199 (Fc-CH₂, 31 %); 121 (FeCp, 40%)

### c) 5-Ferrocenylpentansäureethylester (III)

Zu 1 g (3,5 mmol) 5-Ferrocenylpentansäure (II) und 500 µl Ethanol in THF wurde 1 g (4,8 mmol) Dicyclohexylcarbodiimid gegeben und über Nacht bei Raumtemperatur gerührt. Am nächsten Tag wurde der ausgefallene Dicyclohexylharnstoff abgesaugt, der Reaktionsansatz eingeengt und ohne weiteres Aufarbeiten chromatographiert.

Ausbeute: 692 mg (63 % d. Th.)
DC: R_{f} (Kieselgel, CHCl₃); 0,8
SC: Alox, neutral (Akt.III), PE/EE 9:1
¹H-NMR (250 MHz) CDCl₃: δ= 4,17-4,10 (q, 2H, O-CH₂); 4,08 (s, 5H, CP unsubst.); 4,04-4,03 (m, 4H, Cp subst.); 2,37-2,28 (m, 4H, Fc-CH₂, CH₂-COO); 1,72-1,51 (m, 4H, 2x CH₂)
MS: (C₁₇H₂₂FeO₂, 314,09), m/z = 314 (M⁺, 32,6%); 206 (M⁺-Cp-OEt, 49,2 %); 163 (FeCp-CH₂-CH=CH₂, 83,6%), 55 (C₄H₇, 100 %)

### d) 5-Ferrocenylpentanol (IV)

Zu 0,5 g (160 mmol) 5-Ferrocenylpentansäureethylester (III) in 20 ml THF wurden 61 mg (1,60 mmol) LiAlH₄, gegeben und 2 h bei RT gerührt. Der Ansatz wurde hydrolysiert, es wurden 50 ml Dichlormethan zugegeben, mit 2 molarer Salzsäure neutralisiert und dreimal mit H₂O gewaschen, mit NaSO₄ getrocknet und eingeengt.

Ausbeute: 378 mg (89 % d.Th.)
DC: R,(Alox, CH₂Cl₂) = 0,2
SC: Alox, neutr. (Akt.III), CH₂Cl₂
₁H-NMR (250 MHz) CDCl₃: δ =4,08 (s, 5H, Cp unsubst.); 4,10-4,02 (m, 4H, Cp subst.); 3,68-3,61 (dt, 2H, CH₂-OH); 2,37-2,31 (m, 2H, Fc-CH₂), 1,65-1,49 (m, 4H, 2x CH₂); 1,45-1,35 (m, 2H, CH₂)
MS: (C₁₅H₂₀FeO, 272,08), m/z = 272 (M⁺, 100%); 199 (Fc-CH₂, 20,2 %); 121 (FeCp, 22,4 %)

### e) (5-Ferrocenylpentyl)-(2-cyanoethyl)-diisopropylamidophosphit (VI)

0,150 g (0,55 mmol) des 5-Ferrocenylpentanols (IV) wurden zweimal mit THF eingeengt, mit 5ml THF aufgenommen und 376 µl (2,2 mmol) Hünig-Base zugefügt. Zu dieser Reaktionsmischung wurden 195 µl (0,83 mmol) Chloro-N,N-diisopropylamino-cyanoethoxyphosphin (V) mit Hilfe einer Spritze über ein Septum zugetropft. Nach 15 min bei RT wurden 50 µl H₂O zugegeben und weitere 30 Minuten gerührt. Der Ansatz wurde in 20 ml EE/NEt₃ aufgenommen und mit 10 % NaHCO₃-Lösung ausgeschüttelt, anschließend 2 x mit Wasser gewaschen, mit NaSO₄ getrocknet und eingeengt.

Ausbeute: 164 mg (94 % d.Th.)
DC: R_{f}(Alox, CH₂Cl₂) = 0,61
SC: KG, PE/EE/NEt₃ 18:1:1
¹H-NMR (250 MHz) CDCl₃: δ = 4,08 (s, 5H, Cp unsubst.); 4,04-4,02 (m, 4H, Cp subst.); 3,89-3,75 (2x sep, je 1H, NCHMe₂); 3,70-3,52 (m, 4H, CH₂-Cyanoethyl, 5"-H₂); 2,65-2,60 (t, 2H, CH₂-CN); 2,36-2,30 (m, 2H, 1"-H₂); 1,68-1,35 (m, 6H, 3x CH₂); 1,20-1,16 (4x s, je 3H, 4x CH₃)
MS: (C₂₄H₃₇FeN₂ O₂P, 472,19), m/z = 472 (M⁺, 14,4 %); 389 (M⁺-N((CH(CH₃)₂-CN, 63,0 %); 121 (FeCp, 100 %)

### Beispiel 2: Markierung von Oligonukleotiden mit Ferrocen

### a) (5-Ferrocenylpentyl)-(2-cyanoethyl)-(3'-O-acetyl-2'-desoxythymidin-5')-phosphittriester (VII)

50 mg (0,18 mmol) 3'-O-Acetyl-2'-desoxythymidin und 20 mg (0,29 mmol) Tetrazol wurden zweimal mit 5 ml wasserfreiem CH₃CN eingeengt und mit 5 ml CH₃CN aufgenommen. Dazu wurden langsam 100 mg (0,21 mmol) des Phosphoramidites (VI) in 10 ml CH₃CN getropft, welches zuvor ebenfalls zweimal mit CH₃CN eingeengt worden war. Nach einer Stunde Rühren bei RT wurden dem Ansatz 100 ml PE/EE 9:1 zugefügt, mit 10 % NaHCO₃Lösung, 2x mit H₂O gewaschen und eingeengt.

Ausbeute: 103 mg (88 % d.Th.)
DC: R_{f}(KG, CHCl₃/MeOH 95/5) = 0,5
SC: KG, CHCl₃/MeOH/NEt₃ 98/1/1

### b) Triethylammonium-(5-ferrocenylpentyl)-(3'-O-acetyl-2'-desoxythymidin-5')-phosphat (VIII)

103 mg (0,15 mmol) des Phosphittriesters (VII) wurden mit 1 ml einer 5 % Cumolhydroperoxid-Lösung in CH₃CN versetzt. Nach 3 min wurde die Reaktion durch die Zugabe von 100µl Isopropanol gestoppt. Der Ansatz wurde mit 1 ml Triethylamin versetzt und ohne weiteres Aufarbeiten vorsichtig eingeengt und sofort chromatographiert. Die Cyanoethoxy-Gruppe wird dabei durch das Triethylanim abgespalten.

Ausbeute: 104 mg (93 % d.Th.)
DC: R_{f}(KG, CHCl₃/MeOH 95/5) = 0,2
SC: KG, CHCl₃/MeOH/NEt₃ 98 1/1
¹H-NMR (250 MHz) CDCl₃: δ = 7,77 (q, 1H, 6-H); 6,29-6,24 (dd, 1H; 1'-H); 5,29-5,28 (dt, 1H, 3'-H); 4,12-4,08 (m, 2H, Cp subst.); 4,06 (s, 5H, Cp unsubst.); 4,05-3,98 (m, 5H, Cp subst., 4'H, 5'H₂); 3,84-3,76 (dt, 2H, 5"-H₂); 2,32-2,22 (m, 4H, 2'-H, 1"-H₂); 2,03 (s, 3H, C(O)CH₃); 1,84 (d, 3H, 5-CH₃); 1,66-1,42 (m, 6H, 3xCH₂) J_{5-Me,6} = 1,18; J_{1',2'} = 6,53; J_{5',P} = 6,45; J_{6",P} = 6,45

### c) Triethylammonium-(5-ferrocenylpentyl)-2'-desoxythymidin-5')-phosphat (IX)

Der Phosphatdiester (VIII) wurde mit 1 ml MeOH/H₂O/NEt₃ 4:2:1 versetzt. Nach einer halben Stunde wurde der Ansatz eingeengt, zweimal mit Ethanol aufgenommen und erneut eingeengt.

¹H-NMR (250 MHz) CDCl₃: δ = 7,66 (q, 1H, 6-H); 6,21-6,16 (dd, 1H, 1'-H); 4,40-4,35 (m, 1H, 3'-H); 4,22-4,20 (dd, 2H, Cp subst.); 4,10-3,87 (m, 7H, Cp subst., 4'H, 5'-H₂); 4,06 (s, 5H, Cp unsubst.); 3,81-3,73 (dt, 2H,6"-H₂); 2,33-2,27 (m, 2H, 1"-H₂); 2,18-2,11 (m, 2H, 2'-H); 1,85 (d, 3H, 5-CH₃); 1,66-1,44 (m, 4H, 2xCH₂); 1,43-1,28 (m, 2H, CH₂)
J_{5-Me,6} = 1,22; J_{1',2'} = 6,55; J_{5',P} = 6,53

### Beispiel 3: Allgemeine Vorschrift zur Synthese der Triethylammonium-(5-ferrocenylpentyl)-(2'-desoxynukleosid-5')-phosphate an der Festphase

Die Säulen mit der Festphase enthalten in der Regel 0,2 µmol eines Nukleosides. In diesem Fall jedoch wurden Festphasen mit der Beladung 1µmol gewählt, um eine spektroskopische Untersuchung zu ermöglichen. Die Säulen die in der automatisierten Oligonukleotidsynthese verwendet werden, sind so beschaffen, daß man auf beiden Seiten eine Luer-Spritze aufsetzen kann. Man kann so die Säule mit den gewünschten Reaktionspartnern oder Lösungsmitteln spülen, indem man sie von einer Spritze in die andere drückt. Die Festphasensynthese wurde mit allen vier Desoxynukleosiden durchgeführt:
1. Abspalten der Dimethoxytritylschutzgruppe: 2x je 1 ml 3% Dichloressigsäure in Dichlormethan für 1 min
2. Spülen mit dem Abspaltungsreagenz bis das Eluat farblos ist
3. Spülen mit mindestens 5x1 ml Dichlormethan, zum Entfernen jeglicher Säurespuren
4. Restliches Lösungsmittel an der Wasserstrahlpumpe bis zur Trockene abziehen
5. Belüften mit Argon
6. Kopplung: 180 µl einer Tetrazol-Lösung (35 mg/ml) in Acetonitril und 23,6 mg (50 µmol) des Phosphoramidites (VI) in 500 µl Acetonitril werden gemischt und sofort auf die Festphase gegeben. Nach 3 min wird das Reaktionsgemisch grob ausgespült und der Vorgang wiederholt.
7. Spülen mit mindestens 5x 1 ml Acetonitril
8. Oxidation: 1 ml einer 5% Cumolhydroperoxid in Acetonitril für 2 min
9. Spülen mit mindestens 5x 1 ml Acetonitril
10. Abziehen des restlichen Lösungsmittels an der Wasserstrahlpumpe
11. Festphase mit 1 ml einer konzentrierten Ammoniak/Methylamin-Lösung (jeweils ca. 10-30%) 5 min inkubieren
12. Lösung in ein verschraubbares Eppendorf-Reaktionsgefäß spülen und für 1 h bei 55°C inkubieren.

Anschließend wird der Reaktionsansatz am Lyophilisator und zur Weiterverarbeitung mit 1 ml TEAA/CH₃CN 1:1 aufgelöst.

| Verbindung | λₘₐₓ Fc-dN [nm] | λₘₐₓ dNMP¹[nm] | Rt-Zeit²[min] | OD | Ausbeute [%] |
|---|---|---|---|---|---|
| Fc-dT | 264 | 267 | 33 | 2,28 | 23,7 |
| Fc-dC | 270 | 271 | 31 | 2,78 | 30,5 |
| Fc-dG | 252 | 252 | 24 | 3,85 | 28,8 |
| Fc-dA | 257 | 259 | 34 | 2,17 | 14,1 |

| | | | | | |
|---|---|---|---|---|---|
| ¹nach Maniatis, Sambrook, 1989 | | | | | |
| ²Die HPLC wurde auf einer präparativen RP₁₈-Säule durchgeführt. Fluß 4 ml/min, 25 %-75% B in 40 min, A: 90 % TEAA/10 % CH₃CN,B: 10 % TEAA/90 % CH₃CN | | | | | |

Die Absoprtion des Ferrocens ist bei dieser geringen Menge zu vernachlässigen. Zur Berechnung der Ausbeute wurden die Absorbtionswerte für die entsprechenden dNMPs herangezogen.

### Triethylammonium-(5-ferrocenylpentyl)-(2'-desoxythymidin-5')-phosphat

MS (ESI): (C₂₅H₃₃FeN₂O₃P, 576,16): m/z = (575,1 [M-H]); (449,0 [M-Thymin-H]⁻); (383,0 [M-Thymin-Cp-H]); (351,1 [Fc-(CH₂)₅-O-PO₃]); (285,1 (CpFe-(CH₂)₅-O-PO₃]; (125,2 [Thymin-H])

**Triethylammonium-(5-ferrocenylpentyl)-(2'-desoxycytidin-5')-phosphat** MS (ESI): (C₂₄H₃₂FeN₃O₇P, 561,13): m/z = (560,1 [M-H]);

**Triethylammonium-(5-ferrocenylpentyl)-(2'-desoxyguanosin-5')-phosphat** MS (ESI): (C₂₅H₃₂FeN₅O₇P, 601,14): m/z = (600,1 [M-H]);

**Triethylammonium-(5-ferrocenylpentyl)-(2'-desoxyadenosin-5')-phosphat**
MS (ESI): (C₂₅H₃₂FeN₅O₆P, 585,14): m/z = (584,1 [M-H]⁻); (448,8, [M-Adenin-H]⁻); (383,1 [M-Adenin-Cp-H]⁻); (350,9 [Fc-(CH₂)₅-O-PO₃]⁻) (285,1 [CpFe-(CH₂)₅-O-PO₃]⁻); (134,2 [Adenin-H]⁻) (586,0 [M+H]⁺) (352,1 [Fc-(CH₂)₅-O-PO₃+H]⁺); (136,2 [Adenin+H]⁺)

### Ferrocenylverknüpftes 4-mer

Die Seqeuenz ^{5'}TGAC^{3'} wurde mit der automatisierten Oligonukleotidsynthese (1µmol) hergestellt. Die Dimethoxytrityl-Gruppe am Ende des letzten Nukleotids wurde dann von Hand abgespalten und weiterhin so verarbeitet wie auch die Monomere. Die präparative HPLC wurde unter geänderten Bedingungen durchgeführt.

HPLC: RP₁₈-Säule, Fluß 4 ml/min, 10 %-50 % B in 40 min und 10 min bei 50 %, A: 90 % TEAA/10 % CH₃CN, B: 10 % TEAA/ 90 % CH₃CN
Retentionszeit 4-mer: 43 min
MS (ESI): (C₅₄H₆₉FeN₁₅O₂₅P₄₉ 1507,28): m/z = (1506,9 [M-H]; (1064,5 [M-Cytidin-CpFe-OH]); (619,0 [pApC])

### Ferrocenylverknüpftes 27-mer

Das 27-mer mit der Sequenz 5'TTC CTC CTT CCT TCC TTC CTT CCT CCC3' wurde am Oligonukleotidsynthesizer hergestellt und das Ferrocen mit der Hand analog zu den Monomeren und dem 4-mer addiert.

Die Analytik mit Hilfe der HPLC lief unter den gleichen Bedingungen wie bei dem 4-mer:
Retentionszeit Ferrocenylverknüpftes 27-mer: 35,3 min

## Patentansprüche

1. Verwendung eines Metallocen-Phosphoramidit-Konjugats, das ein oder mehrere Metallocene und ein oder mehrere Phosphoramidite aufweist, zur elektronenmikroskopisch detektierbaren Markierung von Oligonukleotiden bzw. DNA bzw. RNA, wobei die Bildung eines Metallocen-Phosphat-Oligonukleotid- bzw. Metallocen-Phosphat-DNA- bzw. Metallocen-Phosphat-RNA-Konjugats erfolgt.

2. Verwendung nach Anspruch 1, **dadurch gekennzeichnet, daß** das oder die Metallocene über einen Spacer an das oder die Phosphoramidite gebunden sind.

3. Verwendung nach Anspruch 1 oder 2, **dadurch gekennzeichnet, daß** das Metallocen ein Übergangsmetall-Metallocen ist.

4. Verwendung nach Anspruch 3, **dadurch gekennzeichnet, daß** das Metallocen Ferrocen, Ruthenocen oder Osmocen ist.

5. Verwendung nach einem der Ansprüche 2-4, **dadurch gekennzeichnet, daß** der Spacer eine C₁ - C₁₀ Alkylgruppe ist.

6. Verwendung nach einem der Ansprüche 1 bis 5, **dadurch gekennzeichnet, daß** die in dem Konjugat vorhandenen Metallocene gleich oder verschieden sind.

7. Verwendung nach einem der.Ansprüche 1 bis 6, **dadurch gekennzeichnet, daß** der Phosphoramidit-Bestandteil aus der Verbindung Chloco-N,N-diisopropylamino-cyanoethoxyphosphit stammt.

8. Verwendung nach Anspruch 7, **dadurch gekennzeichnet, daß** es sich bei dem Metallocen-Phosphoramidit-KonjugatumMetallocenylalkyl-(2-cyanoethyl)-diisoproylamidophosphit handelt, wobei das Metall ein Übergangsmetall, insbesondere Eisen, Ruthenium oder Osmium ist.

9. Verwendung nach einem der Ansprüche 1-8, **dadurch gekennzeichnet, daß** die Markierung während der Oligonukleotidsynthese stattfindet, wobei diese unter oxidativen Bedingungen stattfindet, so daß der Phosphoramiditbestandteil zu einer Phosphatgruppe wird.

## Claims

1. Use of a metallocene phosphoramidite conjugate comprising one or more metallocenes and one or more phosphoramidites for the electron-microscopically detectable labeling of oligonucleotides or DNA or RNA, wherein a metallocene phosphate oligonucleotide conjugate or metallocene phosphate DNA conjugate or metallocene phosphate RNA conjugate is formed.

2. Use according to claim 1, **characterized in that** the metallocene or metallocenes are bound to the phosphoramidite or phosphoramidites via a spacer.

3. Use according to claim 1 or 2, **characterized in that** the metallocene is a transitional metal metallocene.

4. Use according to claim 3, **characterized in that** the metallocene is ferrocene, ruthenocene, or osmocene.

5. Use according to any of claims 2 to 4, **characterized in that** the spacer is a C₁-C₁₀ alkyl group.

6. Use according to any of claims 1 to 5, **characterized in that** the metallocenes present in the conjugate are equal or different.

7. Use according to any of claims 1 to 6, **characterized in that** the phosphoramidite constituent originates from the compound chloro-N,N-diisopropylamino cyanoethoxyphosphite.

8. Use according to claim 7, **characterized in that** the metallocene phosphoramidite conjugate is metallocenylalkyl-(2-cyanoethyl)-diisopropylamidophosphite, the metal being a transitional metal, in particular iron, ruthenium or osmium.

9. Use according to any of claims 1 to 8, **characterized in that** labeling is carried out during the oligonucleotide synthesis which takes place under oxidative conditions so that the phosphoramidite constituent becomes a phosphate group.

## Revendications

1. Utilisation d'un conjugué métallocène-phosphoramidite, qui présente un ou plusieurs métallocènes et un ou plusieurs phosphoramidites, pour le marquage détectable au microscope électronique d'oligonucléotides ou d'ADN ou d'ARN, duquel il résulte la formation d'un conjugué métallocène-phosphate-oligonucléotide ou métallocène-phosphate-ADN ou métallocène-phosphate-ARN.

2. Utilisation suivant la revendication 1, **caractérisée en ce que** le ou les métallocènes sont liés au phosphoramidite ou aux phosphoramidites par l'intermédiaire d'un espaceur.

3. Utilisation suivant la revendication 1 ou 2, **caractérisée en ce que** le métallocène est un métallocène de métal de transition.

4. Utilisation suivant la revendication 3, **caractérisée en ce que** le métallocène est un ferrocène, un ruthénocène ou un osmocène.

5. Utilisation suivant l'une des revendications 2 à 4, **caractérisée en ce que** l'espaceur est un groupe alkyle en C₁ à C₁₀.

6. Utilisation suivant l'une des revendications 1 à 5, **caractérisée en ce que** les métallocènes présents dans le conjugué sont identiques ou différents.

7. Utilisation suivant l'une des revendications 1 à 6, **caractérisée en ce que** le constituant phosphoramidite provient du composé chloro-N,N-diisopropylamino-cyanéthoxy-phosphite.

8. Utilisation suivant la revendication 7, **caractérisée en ce que** le conjugué métallocène-phosphoramidite consiste en un métallocénylalkyl-(2-cyanéthyl)-diisopropylamidophosphite, dans lequel le métal est un métal de transition, en particulier le fer, le ruthénium ou l'osmium.

9. Utilisation suivant l'une des revendications 1 à 8, **caractérisée en ce que** le marquage a lieu pendant la synthèse de l'oligonucléotide, qui est conduite dans des conditions oxydantes, de telle sorte que le constituant phosphoramidite devient un groupe phosphate.
